# EUROPEAN PATENT APPLICATION

(11) **EP 4 289 337 A1**
(43) Date of publication of application: **13.12.2023**
(21) Application number: 22382585.2
(22) Date of filing: 20.06.2022
(51) Int. Cl.: A61B 3/08

(54) **BINOCULAR SEE-THROUGH VISION DEVICE FOR FAR AND NEAR DISTANCES**

(30) Priority: 08.06.2022 EP 22382550
(71) Applicant: 2Eyes Vision, S.L., 28760 Tres Cantos (ES); Consejo Superior de Investigaciones Cientificas, 28006 Madrid (ES)
(72) Inventor: Gambra Urralburu, Enrique, 28760 Tres Cantos (ES); Dorronsoro Díaz, Carlos, 28760 Tres Cantos (ES); Barcala Gosende, Xoana, 28760 Tres Cantos (ES); Sisó Fuertes, Irene, 28760 Tres Cantos (ES); Alonso Sanz, José Ramón, 28760 Tres Cantos (ES); Urizar Ursua, María Pilar, 28760 Tres Cantos (ES); Esteban Ibáñez, Eduardo, 28760 Tres Cantos (ES); Rodríguez López, Víctor, 28006 Madrid (ES); Marcos Celestino, Susana, 28006 Madrid (ES)
(74) Representative: Elion IP, S.L.

(57) **Abstract**

The present disclosure relates to a see-through binocular device (100) for far, intermediate and near distances, which comprises two see-through optical modules (110a, 110b), each see-through optical module (110a, 110b) configured for projecting without inversion an image (1a, 1b, 1c; 2a, 2b, 2c) of an object (1, 2) on a first plane each see-through optical module (110a, 110b) comprising:
i. an optical element with variable optical power (120); and
ii. an optical projection system (130) configured for projecting the optical element with variable optical power (120) on a second plane (120');
wherein:
- the input optical axes (113a, 113b) of the two see-through optical modules (110a, 110b) intersect at a plane corresponding to a near distance, such that their fields of view (112a, 112b) overlap completely or almost completely for viewing objects located at a near distance.

The disclosure also relates to a system and method for determining sensory eye dominance and/or for determining sensory eye dominance strength.

## Description

The present disclosure is encompassed within the field of visual optics.

### BACKGROUND

Conventional binoculars are normally used only for far distance vision -usually distances of several meters-, and they are commonly used to provide a significant magnification of the objects located at that far distance. Typically, the range of magnification of conventional binoculars is from x7 to x12 times. The field of view (which is the area of the object space that can be seen through an optical system) also typically decreases proportionally with the magnification factor: the larger the magnification, the smaller the field of view. In fact, binoculars are not intended for seeing objects located at intermediate and near distances. The definition of far, intermediate and near distance is not uniform in the field, but a good reference that will be used in the present document is to consider far distance anything beyond 2 m (up to infinity); intermediate distance, between 2 m and 50 cm; and near distance, between 25 cm and 50 cm. This reference regarding distances has been taken from Buckhurst P. J. et al, "Multifocal Intraocular Lens Differentiation Using Defocus Curves", Investigative Ophthalmology & Visual Science, June 2012, Vol. 53, No. 7.

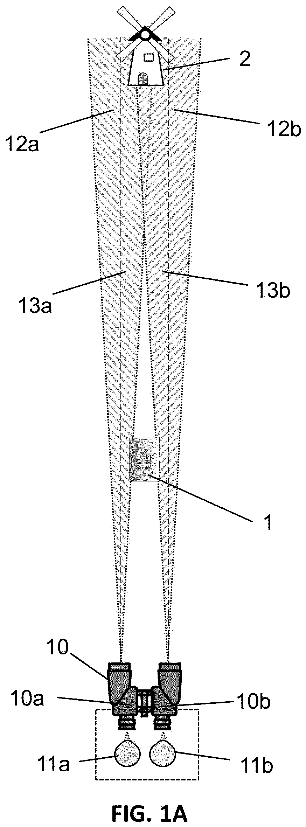

Figure 1A depicts a pair of binoculars 10 used by a user, whose left and right eyes 11a, 11b are schematically represented. The binoculars 10 are used for seeing an object 1 located at a certain distance; the object 1 in the example shown is a book of Don Quixote, which is located at a distance of around 40 cm from the position of the user. The binoculars 10 are also used for seeing another object 2 located at a far distance; the object 2 in this case is a windmill located at around 100 m from the user. The field of view produced by the left optical channel 10a of the binocular is represented by the striped area 12a, and the field of view produced by the right channel 10b of the binocular is represented by the striped area 12b.

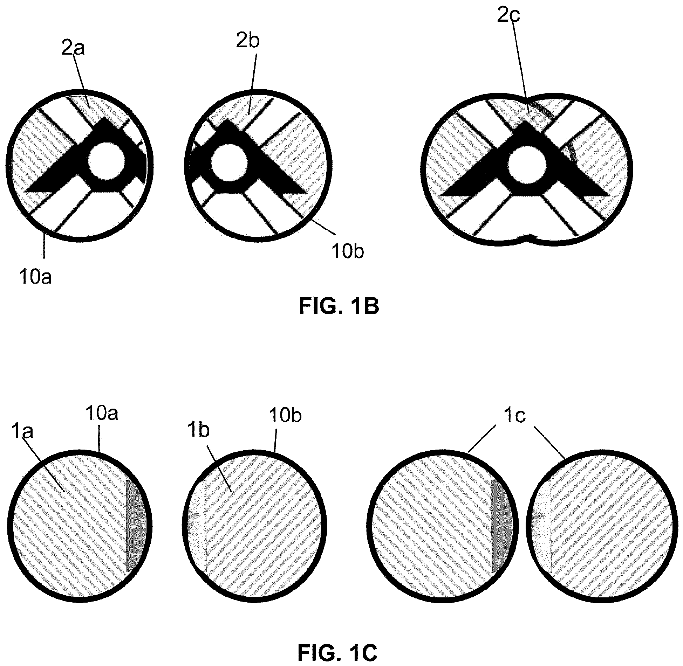

The left-hand side portion of Figure 1B shows the images 2a, 2b actually perceived by the user of the object 2 located at a far distance (i.e., the windmill), through the left optical channel 10a and the right optical channel 10b of the binoculars, respectively, which are zoomed-in portions of the object 2 (in the example shown, the user views portions of the windmill blades) with a slightly different point of view. Since the fields of view 12a and 12b overlap at far distances, the two images 2a, 2b viewed by the user with the binoculars significantly overlap, and the image 2c produced by the binocular vision is schematically represented on the right-hand image of Figure 1B.

In a similar way to Figure 1B, the left-hand side portion of Figure 1C shows the images 1a, 1b actually perceived by the user of the object 1 located at a near distance (i.e., the boof of Don Quixote) through the left optical channel 10a and the right optical channel 10b, respectively. But the fields of view 12a and 12b do not overlap at intermediate distances, least at a near distance, and the two images seen by the user with the binoculars do not overlap. As a result, there is no actual binocular vision for the user when viewing the book of Don Quixote through the binoculars; the user actually perceives two separate non-overlapping images 1c of the book of Don Quixote as schematically represented on the right-hand image of Figure 1C.

The fact is that binoculars do not produce binocular vision when viewing objects located at intermediate or near distances. At these distances - typically closer than 2 m-, the fields of view produced by the left and right optical channels are separate; the result is a very uncomfortable vision for the observer. Moreover, in addition to this separation effect, binoculars usually are not capable of focusing images at intermediate and near distances. In fact, binoculars are not intended for seeing objects located at intermediate and near distances.

These undesirable effects become worse when the field of view for each of the left/right optical channels is reduced.

On a separate note, the authors are aware of patent document EP3053512A1, which discloses a miniature simultaneous vision simulator instrument that projects a tunable lens in the plane of the subject's pupil. The instrument disclosed in this patent document allows the simulation of multifocal lenses in a monocular way, using a temporal multiplexing technique if the tunable lens is fast enough. Yet, this instrument does not provide a working solution for binocular vision at near, intermediate and far distances.

Thus, there is a need for a binocular device that overcomes the drawbacks of the existing solutions and permits the user to view objects located at near, intermediate and far distances.

### SUMMARY

The present disclosure intends to solve the shortcomings of prior-art binocular devices by providing a see-through binocular device producing a significant area of overlapping between the field of view of the left and right channels for far, intermediate and near distances, what will provide stereoscopic vision for all distances.

Through the text, the see-through binocular device may also be referred to as binocular device.

An aspect of this disclosure relates to a see-through binocular device for far, intermediate and near distances, the binocular device comprising:
- two see-through optical modules, each see-through module configured for projecting without inversion an image on a first plane, each see-through optical module comprising:
   i. an optical element with variable optical power; and
   ii. an optical projection system configured for projecting the optical element with variable optical power on a second plane;
   wherein:
   - the output optical axis of each see-through optical module may have equal deviation with respect to the input optical axis, which deviation preferably equals zero (that is, there is no deviation between the output axes of the two see-through optical modules), so as not to produce double vision in a user of the binocular device;
   - the input axes of the two see-through optical modules intersect at a plane corresponding to a near distance such that their fields of view overlap completely or almost completely for viewing objects located at a near distance.

The binocular device as just defined provides fields of view -through each optical module- that essentially fully overlap for viewing objects located at a near, thus being separated and crossed for observation of objects located at far distances, where crossed means that the visual field of the right eye for distance is on the left and the visual field of the left eye for distance is on the right.

When the binocular device is in use, the first plane essentially coincides with a plane containing the retina of an eye of a user. And the second plane essentially coincides with a pupil plane of the eye of the user.

In any one of the aspects or embodiments of the present disclosure, the optical element with variable optical power can be an optoadjustable lens.

The optical channel may have a magnification ranging between 0.8 and 1.25, although a magnification around 1 is the preferred option.

In certain embodiments, each optical element with variable optical power is configured to set a focus for observation at near, intermediate, or far distances. Each optical element with variable optical power can operate independently from the other optical element with variable optical power. This allows the binocular device of the present disclosure to simulate ophthalmic corrections, which corrections may be different between eyes.

Additionally or alternatively, the optical element with variable optical power can be configured to produce multifocal simultaneous vision by temporal multiplexing.

Accordingly, the binocular device of the present disclosure allows a user thereof to experience different corrections in each eye, something that is increasingly common in presbyopia corrections:
- monovision: a different monofocal correction in each eye, one corrected for distance (usually, the dominant eye) and one for near (usually, the non-dominant eye);
- modified monovision: one eye with a monofocal correction for distance vision (usually, the dominant eye) and the other eye with a multifocal correction (usually, the non-dominant eye); or,
- mix-and-match: different multifocal corrections in each eye.

In certain embodiments each projection system comprises a first and a second groups of lenses having equal focal length F, the first and second groups of lenses being similar, preferably identical, but in inverted in orientation with respect to each other, the first group of lenses being placed at a distance F with respect to a principal plane of the optical element with variable optical power and a principal plane of the second group of lenses being positioned at a distance 2F with respect to the principal plane of the first group of lenses. Thus, a conjugate image of the optical element with variable optical power with unity magnification is generated at the distance F behind the second group of lenses and the projection system comprising the two groups of lenses acts as an afocal system, meaning that it does not modify the vergence of the rays introduced by the optical element with variable optical power.

In certain embodiments, the projection system comprises at least four mirrors for undoing the inversion introduced by the first and second groups of lenses. It is also possible to invert the image produced by the first and second groups of lenses by providing a prism or set of prisms in the projection system. It is also possible to generate a non-inverted image with at least a third group of lenses.

In certain embodiments, each projection system comprises at least one block of free-form optics used in combination, or not, with other groups of lenses and/or mirrors and/or prisms to generate a non-inverted image with unity magnification of the optical element with variable optical power.

In certain embodiments the see-through binocular device comprising an additional offset lens placed next to the optical element with variable optical power. This way, the vergence of the light is additionally modified to move the dioptric range of the binocular device and placed in the desired position. In the context of the present disclosure next is to be understood as comprising within 0 mm and 10 mm; the nearer, the better. The offset lens can be placed before or after -in the direction of the light path- the optical element with variable optical power.

In certain embodiments the distance between the first and second groups of lenses can be varied and be different from 2F. This way the vergence of the light is additionally modified to move the dioptric range of the overall binocular device and placed in the desired position. This embodiment has the potential advantage of being more compact and not needing an extra lens for providing the offset.

The see-through binocular device may comprise a mechanism for adjusting an interpupillary distance. The mechanism is capable of horizontally moving the see-through optical modules without affecting the direction of the optical axes.

In any of the previous embodiments, the see-through binocular device can be configured such that a first distance between the second plane and a certain position -which position essentially coincides with an object to be observed- is equal to a second distance corresponding to the distance traveled by light coming from that certain position up to the principal plane of the optical element with variable optical power after being reflected in a mirror. This way, the real position of the observed object coincides with the position perceived by the user when observed through the binocular device. This may be achieved by providing at least a mirror upstream from the optical element with variable optical power in each see-through optical module.

Another aspect of the present disclosure relates to a method for projecting without inversion an object located at far, intermediate and near distances, the method comprising:
- projecting without inversion the object on a first plane using a first see-through optical module having an optical element with variable optical power, which projecting comprises projecting the optical element with variable optical power on a second plane (PP);
- projecting without inversion the object on the first plane using a second see-through optical module having an optical element with variable optical power, which projecting comprises projecting the optical element with variable optical power on the second plane;
the method further comprising:
- arranging the input optical axes of the two see-through optical modules intersect at a plane corresponding to a near distance.

And, usually,- arranging the output optical axis of each see-through optical module to have equal deviation with respect to the input optical axis of the respective see-through optical module.

According to the above method, two fields of view are generated, which fields of view overlap completely or almost completely for viewing objects located at a near distance.

Additionally or alternatively, the optical element with variable optical power can be configured to produce multifocal simultaneous vision by temporal multiplexing.

Another aspect of the present disclosure relates to a see-through binocular system, the system comprising a see-through binocular device as defined in the foregoing; and an accessory for supporting one or more trial lenses, the accessory and the see-through binocular device comprising attaching means for attaching to each other, in a secure manner so that the trial lenses are centered with respect to the input optical axes of the see-through optical module.

The attachment means may include one or magnets mounted on the accessory and on the see-through binocular device. Or they may comprise plug and socket connectors.

Another aspect of the present disclosure relates to a system for determining sensory eye dominance and/or for determining sensory eye dominance strength, the system comprising:
- a see-through binocular device as defined in any of the previous aspects or embodiments, the binocular device being configured for:
   - carrying out a two-fold test in which an optical power value is introduced in a first one of the optical modules of the see-through binocular device, preferably only in the first one of the optical modules, to produce blur in an image viewed by a user of the see-through binocular device through preferably only one of the optical modules; and afterwards, the optical power value is introduced in the second one of the optical modules, and preferably in only the second one of the optical modules, to produce blur through preferably only the other optical module in the same image viewed by the user;
   - repeating the previous two-fold test a number N of times;
- a user interface for collecting a user's indication during each time of the N times, the user's indication being representative of the user's preference of the image viewed;
- a processor for processing the N user's indications to determine the sensory eye dominance and/or to determine the sensory eye dominance strength.

In the two-fold test, one of the two optical channels is blurred, while the other is left preferably sharp. Each two-fold test can be carried out randomly or pseudo-randomly, so that the optical module that is blurred first can be the one corresponding to the left or the right eye, indistinctly.

In certain embodiments, the number N of times is at least 10. This way, the two-fold test is repeated a reasonable number of times so as to balance the accuracy and reliability of the result, the comfort of the user and the overall duration of the test.

In certain embodiments the optical power value introduced is between 1.0-2.0 D, preferably as a positive optical value.

Another aspect of the present disclosure relates to a method for determining sensory eye dominance and/or for determining sensory eye dominance strength, the method comprises:
- carrying out a two-fold test which comprises:
   - introducing an optical power value in a first optical module of a see-through binocular device, preferably in only the first optical module, to produce blur in an image viewed by a user of the see-through binocular device through preferably only the first optical module, and afterwards,
   - introducing the optical power value in a second optical module of the see-through device, preferably only in the second optical modules, to produce blur in the same image viewed by the user through preferably only the second optical module;
   - repeating the previous two-fold test a number N of times;
- collecting a user's indication during each time of the N times, the user's indication being representative of the user's preference of the image viewed;
- processing the N user's indications to determine the sensory eye dominance and/or to determine the sensory eye dominance strength.

In certain embodiments, the see-through binocular device used for determining sensory eye dominance and/or for determining sensory eye dominance strength is as defined in any of the previous aspects and/or embodiments.

The steps of the computer-implemented method for determining sensory eye dominance and/or for determining sensory eye dominance strength can be performed by a processor. The processor can be an integral part of the binocular device or part of a separate device, such as a laptop, tablet, mobile phone, etc.

The different aspects and embodiments defined in the foregoing may be combined with one another, as long as they are compatible with each other.

Additional advantages and features of the present disclosure will become apparent from the detail description that follows and will be particularly pointed out in the appended claims.

### BRIEF DESCRIPTION OF THE DRAWINGS

To complete the description and in order to provide for a better understanding of the present disclosure, a set of drawings is provided. Said drawings form an integral part of the description and illustrate one or more embodiments, which should not be interpreted as restricting the scope of the disclosure, but just as an example of how the present disclosure can be carried out. The drawings comprise the following figures:
Figure 1A depicts a situation in which a user is viewing with a conventional pair of binoculars two objects, one located at a far distance and another located at an intermediate (or near) distance. Figures 1B and 1C diagrammatically represent the monocular and binocular images viewed by the user at far and near distances, respectively, using conventional binoculars.
Figure 2A shows a similar situation to that of Figure 1A, but using a see-through binocular device according to the present disclosure optimized for viewing an object located at an intermediate distance. And figures 2B and 2C diagrammatically represent the monocular and binocular images viewed by the user at far and near distances, respectively.
Figure 3A shows a similar situation to that of Figure 1A, using a see-through binocular device according to the present disclosure optimized for viewing an object located at near distance. Figures 3B and 3C diagrammatically represent the monocular and binocular images viewed by the user at far and near distances, respectively.
Figure 4A shows a perspective view of an embodiment of a see-through optical module of a binocular device according to the present disclosure. Figures 4B and 4C show top and lateral views, respectively, of the see-through optical modules shown in Figure 4A.
Figure 5 shows a lateral view of another possible embodiment of a see-through optical module.
Figure 6 shows a top view of a see-through binocular device according to another possible embodiment.
Figure 7 shows a lateral view of another possible embodiment of a see-through optical module.

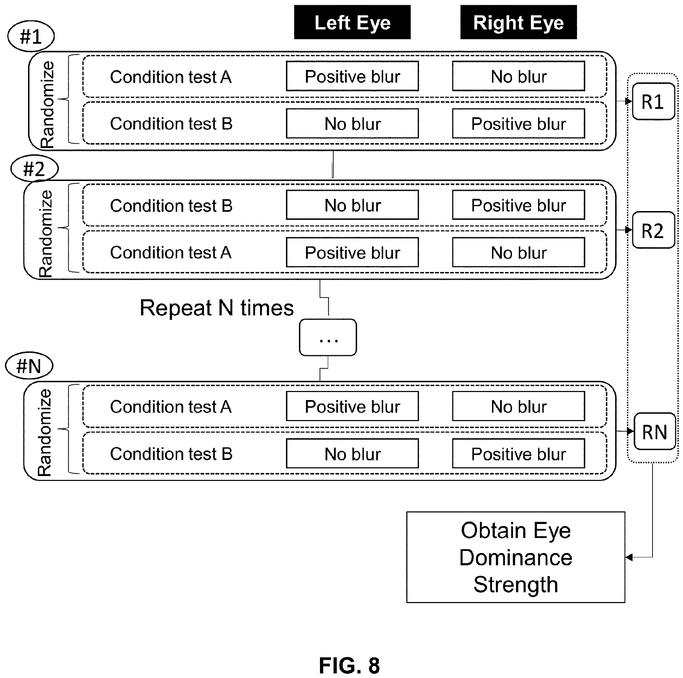

The flow diagram of Figure 8 schematically shows the steps of a method for assessing eye dominance strength according to the present disclosure.

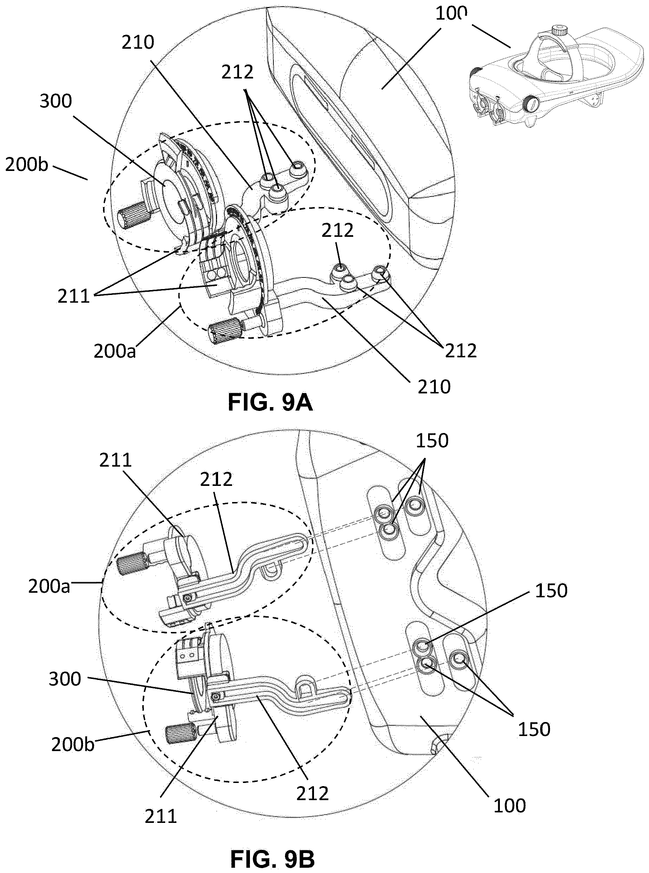

Figure 9A shows a perspective view of a possible embodiment of an accessory for mounting one or more trial lenses on a binocular device according to the present disclosure. Figure 9B shows a bottom view thereof.

### DESCRIPTION OF PREFERRED EMBODIMENTS

The following description is not to be taken in a limiting sense but is given solely for the purpose of describing the broad principles of the invention. Embodiments of the apparatus and method of the present disclosure will be described by way of example, with reference to the accompanying drawings.

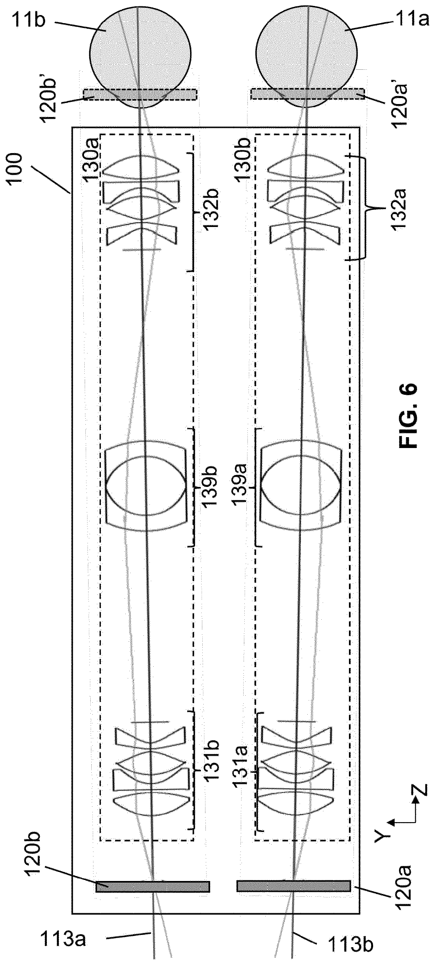

According to the present disclosure, a see-through binocular device 100 for near, intermediatee and far distances is presented, which further allows a user thereof to experience different corrections in each eye. A possible embodiment of the see-through binocular device 100 for near, intermediate, and far distances is schematically represented in Figure 6, and will be explained in detail below.

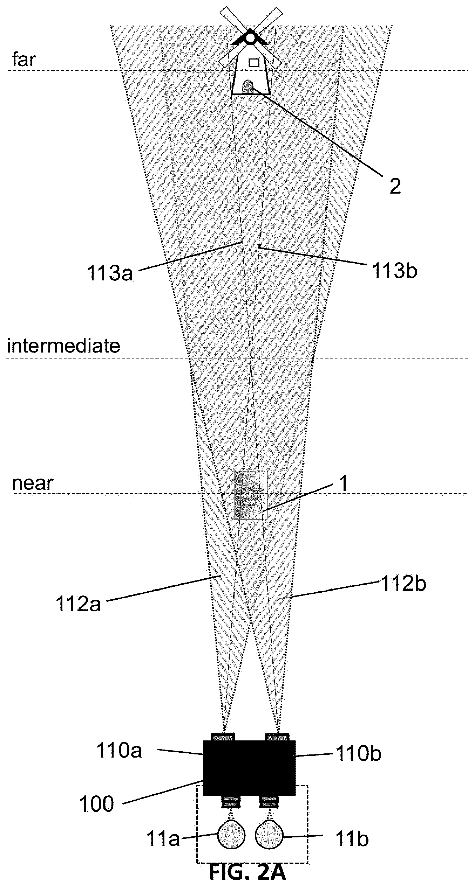

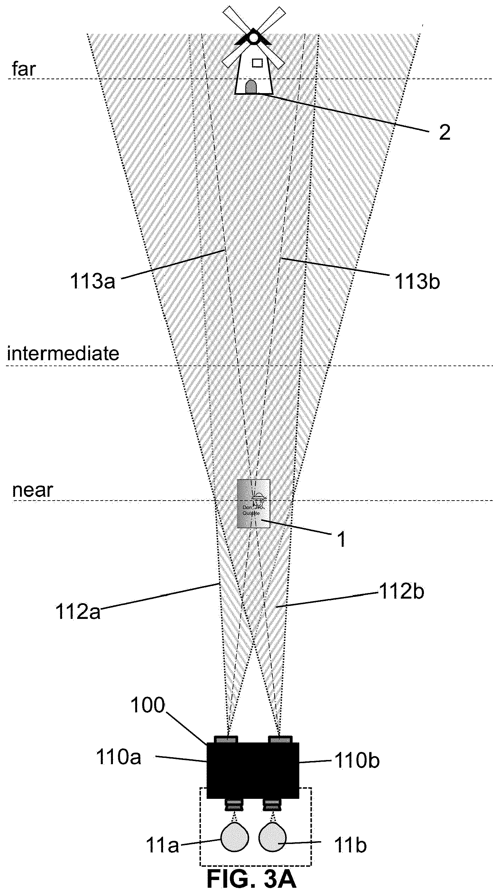

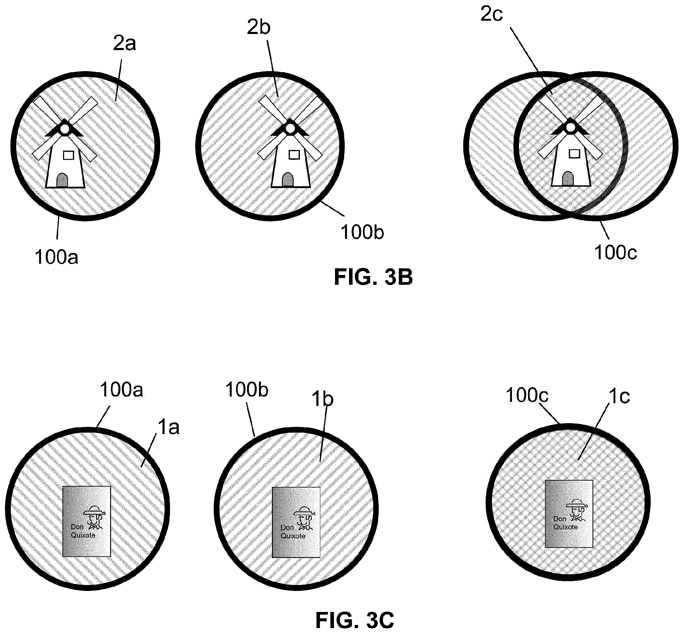

First, the viewing results of the presented see-through binocular device 100 are schematically shown in Figures 2A-2C and 3A-3C.

Figure 2A shows a similar situation to that of Figure 1A, that is, a user (whose left and right eyes 11a, 11b are represented) is presented with a scenario including a book of Don Quixote 1 located at a near distance (of around 40 cm from the user), and a windmill 2 located at a far distance (of around 100 m from the user). The user is viewing this scenario and the objects included therein using a see-through binocular device 100 according to the present disclosure. The binocular device 100 has a left optical channel 110a and a right optical channel 110b. The field of view produced by the left channel 110a of the binocular device 100 is represented by the striped area 112a, and the field of view produced by the right channel 110b of the binocular device 100 is represented by the striped area 112b; the left and right fields of views 112a and 112b totally overlap at the intermediate distance, where the respective input optical axes of the left and right channels 113a and 113b intersect.

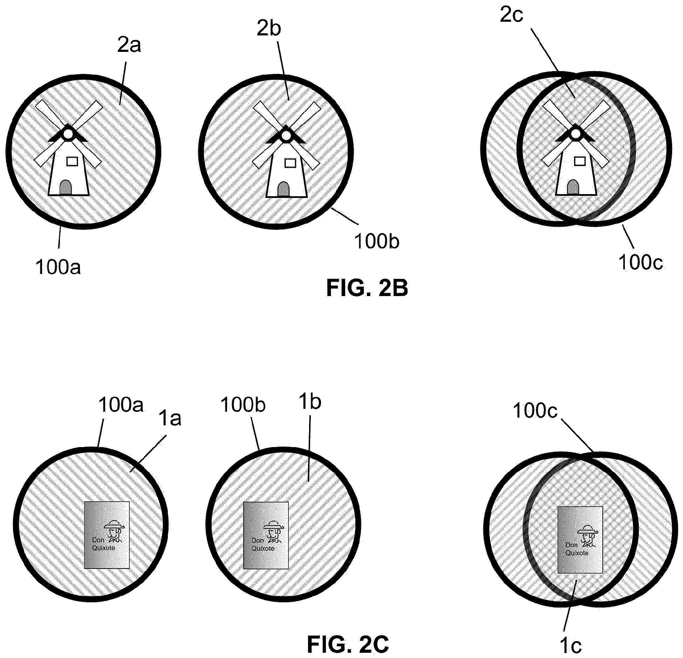

The left-hand side portion of Figure 2B shows the images 2a, 2b actually perceived by the user of the object 2 located at a far distance (i.e., the windmill), through the left and right channels 110a, 110b, respectively, when the binocular device is configured to be optimal for an intermediate distance. In this case, the images 2a, 2b include the windmill 2 (the magnification factor is x1) with a slightly different point of view. Since the fields of view 112a and 112b overlap at far distances, the two images 2a, 2b viewed by the user with the binocular device 100 significantly overlap, and the image 2c produced by the binocular vision is schematically represented on the right-hand image of Figure 2B.

In a similar way to Figure 2B, the left-hand side portion of Figure 2C shows the images 1a, 1b actually perceived by the user of the object 1 located at a near distance (i.e., the book of Don Quixote) through the left and right channels 110a, 110b, respectively, when the binocular device is configured to be optimal for an intermediate distance. The fields of view 112a and 112b do overlap also at near distances, and the two images 1a, 1b viewed by the user with the binocular device 100 also partially overlap. As a result, the user is also able to view the book of Don Quixote 1 through the binocular device 100; the user actually perceives a binocular vision image 1c as schematically represented on the right-hand image of Figure 2C, not two separate images.

In Figure 3A, the see-through binocular device 100 according to the present disclosure is configured for focusing at a near distance, where the book of Don Quixote 2 is. The left and right fields of views 112a and 112b totally overlap at the near distance, where the respective input optical axes of the left and right channels 113a and 113b intersect. Similarly to Figures 1B-1C and 2B-2C, Figures 3B-3C diagrammatically represent the images actually viewed by the user at far and near distances, respectively. Since the binocular device 100 is focusing is configured to be optimal for a near distance, the left and right images 1a, 1b essentially fully overlap to produce a binocular vision image 1c including the book of Don Quixote. And the image 2c of the object located at a far distance, i.e. the windmill, is yet correctly viewed by the user, since the left and right images 2a, 2b partially overlap.

Therefore, the see-through binocular device 100 presented herein satisfactorily resolves the problem posed by the existing solutions, including the instrument disclosed in EP3053512A1.

As a first approach, it may be considered that a balanced situation where the fields are equally overlapped for near and distance objects (and thus perfectly overlapped at a certain intermediate distance, as shown in Figure 2A) would be the best possible scenario. However, several years of experiments carried out internally by the authors of the present disclosure have led the authors to understand that the brain does not tolerate well the separation between visual fields that such configuration would produce for near objects, in which the right field is more to the right and the left field is more to the left; this is disturbing and not tolerable for many subjects. Rather, crossing the fields of view -that is, displacing the right field of view further to the left and the left field of view further to the right- has proven to be well tolerated by the user. Technically this is the situation given by the occlusion of the distant vision by a closer opening: This crossing of fields is like looking through a window.

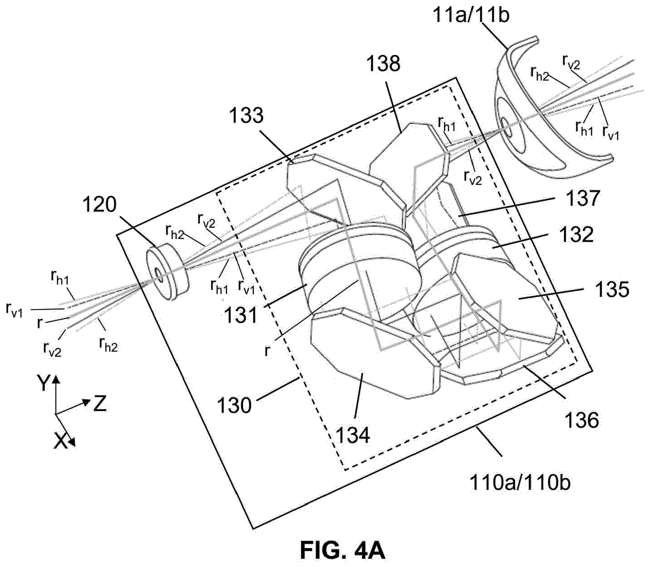

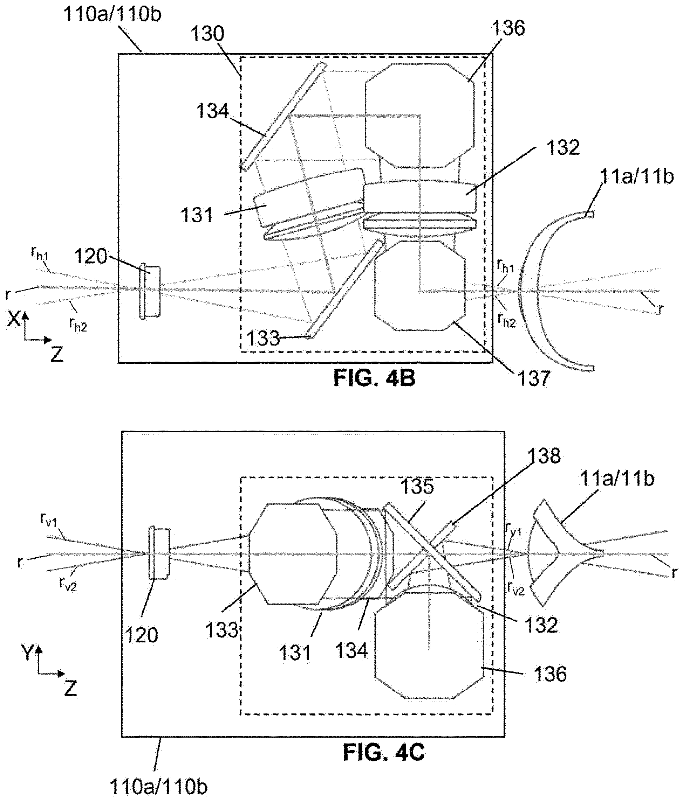

Figure 4 shows a possible embodiment of one of the two see-through optical modules 110a, 110b (also named in the present example as left and right optical channels). The see-through optical module 110a/100b comprises a tunable lens 120 and a projection system 130. The projection system 130 comprises a first and a second groups of lenses 131, 132 and six mirrors 133, 134, 135, 136, 137, 138. The first and second groups of lenses 131, 132 project the tunable lens 120 on the pupil plane of the eye 11a, 11b of the user. Mirrors 135 and 138 invert the image vertically, while mirrors 136 and 137 invert the image horizontally. The combined effect of 131, 132, 135, 136, 137 and 138 produces a non-inverted (or erected) projection. The first two mirrors in the light path, mirrors 133 and 134, are used to obtain the same input and output optical axes.

The path followed by a set of five rays of light within this embodiment of the optical module is schematically shown in Figures 4A, 4B and 4C. The five rays of light are a first ray of light r along the optical axis, two rays of light rᵥ₁ and rᵥ₂ contained in the YZ plane, and two rays of light rₕ₁ and rₕ₂ contained in the XZ plane. It can be appreciated that the disposition of the five rays of light at the entry of the optical module is kept at the entry of the eye lens, there being no inversion; and with the same entry angle, there having unity magnification.

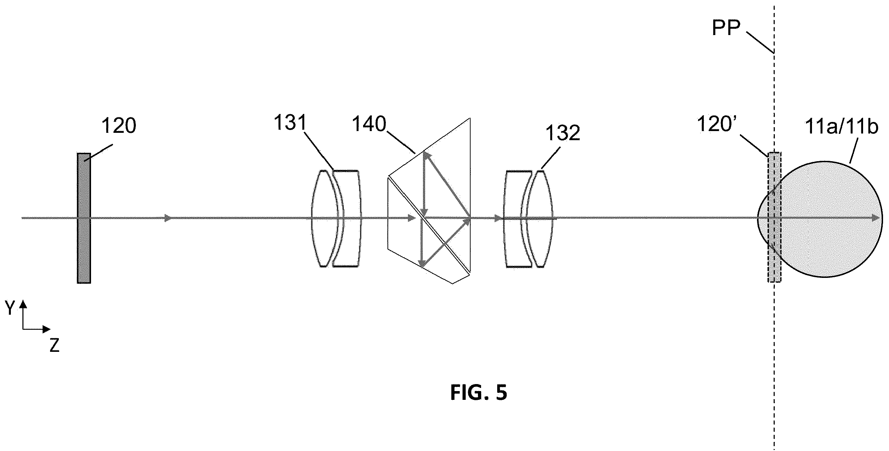

Figure 5 shows another possible embodiment of a see-through optical module in which the optical projection system 130 comprises a first and a second groups of lenses 131, 132 and a group of prisms 140, in this example a Schmidt-Pechan prism is used. The combined effect of the first and second groups of lenses 131, 132 and the prism 140 produces a non-inverted (or erected) projection 120' of a tunable lens 120 on the pupil plane PP of an eye 11a/11b. The path of an input light beam is schematically represented by the arrows.

Figure 6 shows a top view of a see-through binocular device according to another possible embodiment. The see-through binocular device 100 comprises two identical see-through optical modules, including respective identical projection systems 130a, 130b. Each projection system comprises three groups of lenses 131a/131b, 132a/132b and 139a/139b that project respective tunable lenses 120a, 120b with non-inversion on the pupil plane of respective eye 11a, 11b of a user.

Figure 6 represents a binocular device having two optical channels. The embodiments depicted in Figures 4 and 5 just represent one of the optical channels. With any combination of the optical channels depicted in the embodiments of Figures 4, 5 and 6, a binocular device -similar to one described with respect to Figure 6- can be obtained by arranging two optical channels side by side. One of the optical channels is for the left eye 11a and the second optical channel is for the right eye 11b. The two optical channels should be arranged such that the input axes of the two see-through optical modules intersect at a plane corresponding to a near distance; this way, their fields of view overlap completely or almost completely for viewing objects located at a near distance.

In addition, the two optical channels should be arranged at an adjustable distance between them for their alignment with both eyes of the user, in order to match the interpupillary distance of the user.

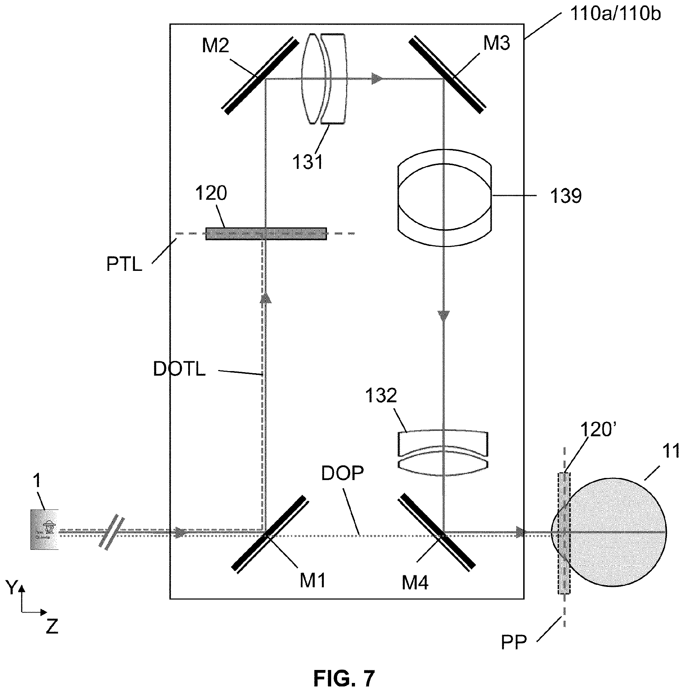

Figure 7 shows a lateral view of another possible embodiment of the see-though optical device, which comprises the following elements: a tunable lens 120, three groups of lenses 131, 132, 139 and four mirrors M1, M2, M3, M4. These elements are arranged such that, when the binocular device is in use, a first distance, DOTL, between the pupil plane PP -where the tunable lens 120 is projected 120'- and an observed object 1 is equal to a second distance, DOP, travelled by light coming from the observed object 1 before reaching the tunable lens 120. This is achieved by inserting the mirror 131 upstream the tunable lens 120, so that the tunable lens 120 is not in front of the eye 11a/11b as is the case of the previous embodiments. Any of the previous embodiments can be modified to achieve this configuration.

Figure 8 schematically shows a diagram flow of a method for determining the sensory eye dominance strength of a person using a see-through binocular device 100 as described in any of the previous embodiments.

According to the proposed method, the see-through binocular device 100 is configured for randomly presenting two test conditions to the user. A first condition -condition test A-implies that the user of the see-through binocular device 100 has no blur in the right eye and a positive blur -for example, 1.5 diopters- in the left eye. Under the second condition -condition test B- the left eye has no blur and a positive blur -for example, 1.5 diopters- is introduced in the right eye. The user of the binocular device 100 is provided with means for indicating which of the first and second conditions prefers during this first two-condition test #1, which preference indication is registered as R1. This means for inputting the user's preference can be implemented by an application, having a user interface; or the user's preference can be collected by means of left/right push buttons. This two-condition test is repeated a second time #2, during which the first and the second condition tests are presented randomly to the user, and the user again provides his/her indication preference, which is register as R2. The text is repeated a number of N times to collect the user's preference each of the N times. After processing the N preference indications of the user, R1, R2... RN, the eye dominance strength for that user is assessed.

For example, if the condition with no blur in the left eye is preferred in 8 out of 10 tests, the user will have left eye dominance with a strength of 80%. Eye dominance strength is calculated as NL/NT or NR/NT (depending on the eye), where NL is the number of times the subject prefers the left eye, NR is the number of times the subject prefers the right eye and NT is the total number of trials.

Figure 9A shows a possible embodiment of an accessory 200a, 200b for mounting one or more trial lenses 300 on a binocular device 100. In fact, Figure 9A shows two embodiments of the accessory: one accessory 200a for mounting onto a left channel of a binocular device 100, and another accessory 200b for mounting onto a right channel of a binocular device 100. Each accessory 200a, 200b comprises an arm 210 having two ends, a first distal end having a support 211 -similar to that of a phoropter- for supporting one or more trial lenses 300, and a second proximal end having a set of magnets 212 (three magnets 212 in the embodiment shown).

The one or more trial lenses 300 can be placed on each support to correct the refractive error (sphere and astigmatism), among other purposes (e.g. introducing trial lenses to obtain a defocus curve). The set of magnets 212 allows easily attaching each accessory 200a, 200b and in a removable manner to the corresponding projector optical system 100, where a corresponding set of three magnets 150 has been arranged (cf. Figure 9B).

The set of magnets 212, 150 are located in such a way that, once the accessory is mounted on the binocular device 100, the trial lenses 300 are centered with respect to the input optical axis of each optical channel. Further, when the interpupillary distance is adjusted in the binocular device 100, the set of magnets 150 is arranged to maintain the alignment of any trial lens 300 eventually mounted on either accessory 200a, 200b.

The present disclosure is obviously not limited to the specific embodiment(s) described herein, but also encompasses any variations that may be considered by any person skilled in the art (for example, as regards the choice of materials, dimensions, components, configuration, etc.), within the general scope of the disclosure as defined in the claims.

In this text, the terms "comprises", "includes" and its derivations (such as "comprising", "including", etc.) should not be understood in an excluding sense, that is, these terms should not be interpreted as excluding the possibility that what is described and defined may include further elements, steps, etc.

## Claims

1. See-through binocular device (100) for far, intermediate and near distances, the binocular device (100) comprising:
- two see-through optical modules (110a, 110b), each see-through optical module (110a, 110b) configured for projecting without inversion an image (1a, 1b, 1c; 2a, 2b, 2c) of an object (1, 2) on a first plane each see-through optical module (110a, 110b) comprising:
i. an optical element with variable optical power (120); and
ii. an optical projection system (130) configured for projecting the optical element with variable optical power (120) on a second plane (120');
wherein:
- the input optical axes (113a, 113b) of the two see-through optical modules (110a, 110b) intersect at a plane corresponding to a near distance, such that their fields of view (112a, 112b) overlap completely or almost completely for viewing objects located at a near distance.

2. The see-through binocular device (100) of claim 1, wherein each optical element with variable optical power (120) is configured to set a focus for observation at near, intermediate, or far distances.

3. The see-through binocular device (100) of any previous claim, wherein the optical element with variable optical power (120) is configured to produce multifocal simultaneous vision by temporal multiplexing.

4. The see-through binocular device (100) of any previous claim, wherein each projection system (130) comprises at least a first and a second groups of lenses (131, 132) having equal focal length F, the first and second groups of lenses (131, 132) being similar, and preferably identical, but in inverted orientations, the first group of lenses (131) being placed at a distance F with respect to a principal plane of the optical element with variable optical power (120) and a principal plane of the second group of lenses (132) being positioned at a distance 2F with respect to the principal plane of the first group of lenses (131).

5. The see-through binocular device (100) of claim 4, wherein each projection system (130) further comprises at least four mirrors (133, 134, 135, 136, 137, 138) for undoing the inversion introduced by the first and second groups of lenses (131, 132).

6. The see-through binocular device (100) of any of claims 4-5, wherein each projection system (130) comprises a prism or set of prisms (140).

7. The see-through binocular device (100) of any of claims 1-6, further comprising means for displacing a dioptric range of the binocular device, by including an additional offset lens placed next to the optical element with variable optical power (120), or by changing the distance between first and second groups of lenses (131, 132).

8. The see-through binocular device (100) of any of claims 1-7, wherein each projection system (130) may comprise at least one block of free-form optics.

9. The see-through binocular device (100) of any preceding claim, wherein each see-through optical module (110a, 110b) comprises at least a mirror (M1), such that a first distance (DOTL) between the second plane (120') and a certain position (1) is equal to a second distance (DOP) corresponding to the distance traveled by light coming from that certain position (1) up to the principal plane of the optical element with variable optical power (120) after being reflected in the mirror (M1).

10. The see-through binocular device (100) of any preceding claim, comprising a mechanism for adjusting an interpupillary distance.

11. Method for projecting without inversion an object located at far, intermediate and near distances, the method comprising:
- projecting without inversion the object (1, 2) on a first plane using a first see-through optical module (110a) having an optical element with variable optical power (120), which projecting comprises projecting the optical element with variable optical power (120) on a second plane (PP);
- projecting without inversion the object (1,2) on the first plane using a second see-through optical module (110b) having an optical element with variable optical power (120), which projecting comprises projecting the optical element with variable optical power (120) on the second plane;
the method further comprising:
- arranging the input optical axes (113a, 113b) of the two see-through optical modules (110a, 110b) to intersect at a plane corresponding to a near distance.

12. A see-through binocular system, comprising:
- see-through binocular device (100) of any of claims 1-10; and,
- an accessory (200a, 200b) for supporting one or more trial lenses (300), wherein the accessory (200a, 200b) and the see-through binocular device (100) comprise attaching means (212, 150) for attaching to each other.

13. System for determining sensory eye dominance and/or for determining sensory eye dominance strength, the apparatus/system comprising:
- a see-through binocular device (100) according to any of claims 1-10, configured for:
- carrying out a two-fold test in which an optical power value is introduced in a first one of the optical modules (110a, 110b) of the see-through binocular device (100) to produce blur through one of the optical modules (110a, 110b) in an image viewed by a user of the see-through binocular device (100), and alternately the optical power value is introduced in the second one of the optical modules (110a, 110b) to produce blur through the other optical module (110a, 110b) in the same image viewed by the user;
- repeating the previous two-fold test a number N of times;
- a user interface for collecting a user's indication during each time of the N times, the user's indication being representative of the user's preference of the image viewed;
- a processor for processing the N user's indications to determine the sensory eye dominance and/or to determine the sensory eye dominance strength.

14. Method for determining sensory eye dominance and/or for determining sensory eye dominance strength, the method comprises:
- carrying out a two-fold test which comprises:
- introducing an optical power value in a first optical module of a see-through binocular device to produce blur through the first optical module in an image viewed by a user of the see-through binocular device, and
- afterwards introducing the optical power value in a second optical module of the see-through device to produce blur through the second optical module in the same image viewed by the user;
- repeating the previous two-fold test a number N of times;
- collecting a user's indication during each time of the N times, the user's indication being representative of the user's preference of the image viewed;
- processing the N user's indications to determine the sensory eye dominance and/or to determine the sensory eye dominance strength.

15. The system of claim 13 or the method of claim 14, where each two-fold test is carried out randomly or pseudo-randomly.

16. The method of any of claims 14-15, wherein the see-through binocular device is according to any of claims 1-10.
